# EUROPÄISCHE PATENTSCHRIFT

(11) **EP 1 581 185 B1**
(45) Veröffentlichungstag und Bekanntmachung des Hinweises auf die Patenterteilung: **14.10.2009**
(21) Anmeldenummer: 03785534.3
(22) Anmeldetag: 24.11.2003
(51) Int. Cl.: A61K 9/127

(54) **LIPOSOMALE GLUCOCORTICOIDE**
LIPOSOMAL GLUCOCORTICOIDS
GLUCOCORTICOIDES LIPOSOMAUX

(30) Priorität: 24.11.2002 DE 10255106
(43) Veröffentlichungstag der Anmeldung: 05.10.2005
(73) Patentinhaber: novosom AG, 06120 Halle (DE)
(72) Erfinder: PANZNER, Steffen, 06144 Halle (DE); BRÄUER, Rolf, 99510 Apolda (DE); KINNE, Raimund, W., 07743 Jena (DE); RAUCHHAUS, Una, 06111 Halle (DE)
(74) Vertreter: Ziebig, Marlene
(86) Internationale Anmeldenummer: PCT/DE2003/003893
(87) Internationale Veröffentlichungsnummer: WO 2004/047792

(56) Entgegenhaltungen:
- EP-A- 1 371 362
- SANDRA SUAREZ; ET AL.: "Effect of Dose and Release Rate on Pulmonary Targeting of Liposomal Triamcinolone Acetonide Phosphate" PHARMACEUTICAL RESEARCH, Bd. 15, Nr. 3, 1998, Seiten 461-465, XP008030350
- J. AL-MUHAMMED; ET AL.: "In-vivo studies on dexamethasone sodium phosphate liposomes" JOURNAL OF MICROENCAPSULATION, Bd. 13, Nr. 3, 1996, Seiten 293-305, XP009000577

## Beschreibung

Die Erfindung betrifft eine liposomale Formulierung umfassend wasserlösliche Glucocorticoide, die Verwendung dieser Formulierung zur Behandlung von inflammatorischen Erkrankungen sowie einen Kit, der die liposomale Formulierung, zum Beispiel als ein pharmazeutisches Mittel, umfasst.

Glucocorticoide sind eine Klasse von Substanzen, die seit langem zur Behandlung von entzündlichen Erkrankungen eingesetzt werden. Die Einarbeitung von Wirkstoffen aus dieser Klasse in liposomale Formulierungen ist konzeptionell bekannt und mehrfach beschrieben. Dabei ist die Formulierung der unmodifizierten Arzneiform schwierig, da liposomal eingeschlossenes Material innerhalb von wenigen Stunden wieder freigesetzt wird. Die bekannten Strategien werden im Folgenden kurz mit Beispielen referiert:
Lipophile Derivatisierung zum Einbau in die Liposomenmembran: Die DE 27 120 30 (ICI 1977) ist ein frühes Beispiel, dass lipophil substituierte Glucocorticoide, beispielsweise Dexamethasonpalmitat, sich stabil in Liposomen einschließen lassen (auch: Mizushima et al. 1982, Yokoyama et al. 1985, Bonanomi et al. 1987). Nachteilhaft an diesen Formulierungen ist der Austausch des hydrophoben Wirkstoffderivats mit Plasmabestandteilen und eine geringe in vivo-Stabilität der Formulierungen.

PEG-Liposomen: Eine Verbesserung demgegenüber wird in der WO 94/07466 (Liposome Technology) offenbart. Durch die Verwendung von PEG (Polyethylenglykol) -modifizierten Lipiden lässt sich die in vivo-Stabilität der Formulierung erhöhen und insbesondere die Zirkulationszeit der Liposomen verlängern. Konzeptionell zielt diese Verbesserung darauf, den unspezifischen Verlust von eingebrachten Liposomen im retikuloendothelialen System zu vermindern und dadurch die spezifische Extravasierung von Liposomen an den Entzündungsherden zu ermöglichen oder zu verbessern.

Wasserlösliche Glucocorticoide können passiv in Liposomen eingeschlossen werden. Derivate wie Phosphatester, Acetonidphosphate und/oder Succinate sind dazu geeignet. Neuere Entwicklungen wie die EP 1 046 394 (Terumo Corp) und die WO 02/45688 (Gerrit Storm) gehen diesen Weg, wobei die wasserlöslichen Glucocorticoide in PEG-modifizierten Liposomen eingschlossen werden (siehe auch: Storm & Richmond 2001, Metselaar et al. 2002, Hussein et al. 2002).

Cyclodextrin-Komplexe mit Glucocorticoiden sind eine andere Möglichkeit, ein wasserlösliches Derivat zu schaffen, welches auf üblichem Weg in Liposomen eingebracht werden kann (Loftsson & Stefansson, 2002, Yano et al. 2002, WO 95/15746 (Gregoriadis)).

Weiterhin sind in der WO 99/15150, der WO 99/55314 und EP 1 190 707 Liposomen offenbart, die wasserlösliche Glucocorticoide enthalten.

Der Stand der Technik hat den Nachteil, dass die erzielten Verbesserungen im Zirkulationsverhalten im Wesentlichen nur bei der erstmaligen Gabe der Formulierung erreicht werden können. Bei mehrmaliger Gabe von PEG-modifizierten Liposomen wird die Bildung von Antikörpern nachgewiesen. Deren Bindung führt bei wiederholter Anwendung zu einer Verringerung der zirkulationshalbwertszeit und ist pharmakologisch unerwünscht (Dams et al. 2000).

Die Aufgabe der Erfindung bestand darin, liposomale Formulierungen von Glucocorticoiden bereitzustellen, die die genannten Nachteile vermeiden und eine vorteilhafte Wirkung gegenüber dem freien Wirkstoff aufweisen.

Die erfindungsgemäße Aufgabe wird gelöst durch die Bereitstellung einer liposomalen Formulierung, wie vorgeschlagen in Anspruch 1.

Über Stunden stabil heißt im Sinne der Erfindung, dass die Formulierung mindestens eine, bevorzugt zwei, besonders bevorzugt drei bis vier Stunden und ganz besonders bevorzugt über fünf bis 24 Stunden stabil im Serum ist.

Überraschenderweise sind die erfindungsgemäßen liposomalen Formulierungen über einen langen Zeitraum im Serum von Organismen anders als die bekannten Strukturen stabil. Das Merkmal der Stabilität ist daher anmeldungsgemäß nicht als Aufgabe an den Fachmann zu verstehen, sondern es dient allein der Klarstellung dahingehend, dass die beschriebenen erfindungsgemäßen liposomalen Formulierungen dieses Merkmal aufweisen. Die erfindungsgemäßen liposomalen Formulierungen bzw. Liposomen sind im Wesentlichen frei von konjugierten Polymeren, insbesondere frei von PEG, PEG-Phosphatidylethanolamin, PEG-Phosphatidylglycerol, PEG-Phosphatidylserin und/oder PEG-Cholesterol bzw. anderen amphiphil modifizierten Polyethylenglykolen.

Überraschend wurde festgestellt, dass liposomale Zubereitungen von Glucocorticoiden, die ohne den Zusatz von PEG-Lipiden hergestellt werden, bei systemischer Anwendung hinreichend stabil sind und eine sehr vorteilhafte Wirkung gegenüber dem freien Glucocorticoid aufweisen, so dass ein besserer therapeutischer Effekt mit einer geringeren Dosis erzielt werden kann. Die erfindungsgemäßen liposomalen Formulierungen, liposomalen Zubereitungen bzw. Liposomen können demgemäß in verschiedenen Bereichen der Diagnose, Prophylaxe und Therapie eingesetzt werden. Die erfindungsgemäßen Strukturen können bevorzugt zur Therapie von entzündlichen Krankheiten eingesetzt werden. Eine bevorzugte Auswahl der Indikationen von Glucocorticoiden ist:
- schwere allergische und anaphylaktische Reaktionen,
- anaphylaktischer Schock,
- Status asthmaticus, schwere Zustände bei Asthma bronchiale,
- Hirnödem, Schädel-Hirn-Trauma, Hirnabszess, bakterielle Meningitis,
- Parenterale Anfangsbehandlung akuter schwerer Hauterkrankungen, (Erythrodermie, Pemphigus vulgaris, akute Ekzeme),
- schwere Infektionserkrankungen, Nebennierenrinden-Insuffizienz,
- idiopathische retroperitoneale Fibrose, Morbus Addison,
- Adrenogenitales Syndrom, chronisch aggressive Hepatitis,
- Lymphogranulomatose, Differentialdiagnose von Morbus Cushing,
- primäre chronische Polyarthritis (rheumatoide Arthritis), chronische Polyarthritis, juvenile Arthritiden (Still-Syndrom), akute rheumatische Karditis, Arthrosen, aktivierte Arthrose, akute Form der Periarthropathia humeroscapularis, nichtbakterielle Tendovaginitis und Bursitis,
- Periarthropathien, Insertionsendopathien, Psoriasis-Arthritis,
- Spondylitis ankylosans (Morbus Bechterew),
- Spondylarthrosen mit Synovitis (aktivierte Arthrose), Spondylosen,
- Riesenzellarteriitis, Morbus Wegener, Churg-Strauß-Syndrom, mikrosk. Polyangiitis,
- nichtinfektiöse Keratokonjunktivitis, Skleritis, Iridozyklitis, Uveitis,
- Kollagenosen (system. Lupus erythematods (vor allem Nephritis), Panarteriitis nodosa, Dermatomyositis, primäres Sjögren-Syndrom),
- akute Schübe von Morbus Crohn, schwere Schübe von Colitis ulcerosa (zum Beispiel toxisches Colon),
- Multibles Myelom,
- PFAFA-Syndrom (periodisches Fieber bei Kindern),
- idiopathische retroperitoneale Fibrose (Morbus Ormond),
- Multiple Sklerose, akute Myasthenia gravis-Krise, angioneurotische Ödeme (zum Beispiel Quincke-Syndrom) und/oder
- akute Alveolitis.

Selbstverständlich ist es möglich, verschiedene wasserlösliche Glucocorticoide einzusetzen. Der Einsatz der verwendeten Glucocorticoide richtet sich insbesondere nach den unterschiedlichen Applikationsrouten. Systemisch applizierte Glucocorticoide sind bevorzugt: Betamethason, Betamethasondihydrogenphosphat-Dinatrium, Budesonid, Cloprednol, Cortison, Cortisonacetat, Deflazacort, Dexamethason, Dexamethasondihydrogenphosphat-Dinatrium, Fluocortolon, Hydrocortison, Methylprednisolon, Prednisolon, Prednison, Prednyliden, Rimexolon und/oder Triamcinolon.

Dermal werden bevorzugt appliziert: Alclometason, Amcinonid, Betamethason, Clobetasol, Clobetason, Clocortolon, Desonid, Desoximethason, Dexamethason, Diflorason, Diflucortolon, Fludroxycortid, Flumethason, Flucinolon, Flucinonid, Fluocortin, Fluocortolon, Fluorometholon, Flupredniden, Fluticason, Halcinonid, Halomethason, Hydocortison, Methylprednisolon, Mometason, Prednicarbat, Prednisolon und/oder Triamcinolon.

Bevorzugt werden im Zusammenhang mit der Erfindung die systemisch applizierten Glucocorticoide, ausgewählt aus obiger Liste, in einer wasserlöslichen Form eingesetzt. Dem Fachmann bekannte wasserlösliche Formen sind Salze und Ester der genannten Wirkstoffe, wie insbesondere die Phosphatester, Sulfatester oder die Dicarbonsäureester oder Additionssalze oder die Glycoside der Wirkstoffe.

Es werden bevorzugt die Mono- oder Disalze eingesetzt, ganz besonders bevorzugt die Natrium- oder Kaliumsalze der Glucocorticoidphosphate, die -acetonidphosphate oder -succinate.

Besonders bevorzugte Verbindungen aus dieser Gruppe sind Betamethasondihydrogenphosphat-Dinatrium, Dexamethasonphosphat, Dexamethasondihydrogenphosphat-Dinatrium, Prednisolonphosphat, Methylprednisolonphosphat, Prednisolonsuccinat, Methylprednisolonsuccinat, Bemethasonphosphat, Desonidphosphat, Hydrocortisonphosphat, Hydrocortisonsuccinat, Prednisolatmahydrochlorid, Prednisonphosphat und/oder Triamcinoloneacetonidphosphat.

Ganz besonders bevorzugte Verbindungen aus dieser Gruppe sind Dexamethasonphosphat, Dexamethasondihydrogenphosphat-Dinatrium und/oder Triamcinoloneacetonidphosphat.

Die bevorzugten erfindungsgemäßen Verbindungen können besonders gut gegen inflammatorische Erkrankungen eingesetzt werden. Der Einsatz der erfindungsgemäßen liposomalen Formulierungen führt zu Vorteilen und Verbesserungen gegenüber den bekannten liposomalen Formulierungen, insbesondere zu einer erhöhten Zuverlässigkeit und Qualitätshebung und größeren Effektivität in der Prophylaxe und Therapie der genannten Erkrankungen. Demgemäß wird durch die erfindungsgemäßen Liposomen der Arzneimittelschatz bereichert und es werden die prophylaktischen und therapeutischen Möglichkeiten der Behandlung verschiedener Erkrankungen vermehrt. Da die erfindungsgemäßen liposomalen Formulierungen eine bessere Wirkung gegenüber dem freien Wirkstoff zeigen, kann dieser in einer niedrigeren Dosis angewendet werden, was zu einem verringerten Nebenwirkungsspektrum führt, ein nicht zu überschätzender Vorteil bei der Therapie mit Glucocorticoiden. Außerdem führt eine Dosisverringerung zu einer Ersparnis an Material, Kosten und schwer zu beschaffenden Feinchemikalien und pharmazeutischen Mitteln. Mit den erfindungsgemäßen Strukturen wird somit ein seit langem ungelöstes dringendes Bedürfnis befriedigt.

Dem Fachmann sind Liposomen bekannt, die er einsetzen muss, um eine möglichst große Serumstabilität zu erreichen und die Bildung von Aggregaten zu verhindern. Besonders bevorzugt werden Liposomen mit einer Größe zwischen 150 nm und 500 nm verwendet.

Liposomen dieser Art sind dem Fachmann bekannt und umfassen auch Liposomen, die aus gesättigten Phospholipiden und Cholesterol aufgebaut sind. In einer bevorzugten Ausführung der erfinderischen Lehre werden zum Aufbau der Liposomen die folgenden Lipide eingesetzt: Dipalmitoylphosphytidylcholin, Distearoylphosphytidylcholin, Dipalmitoylphosphatidylglycerol, Distearoylphosphatidylglycerol, Dipalmitoylphosphatidylserin, Distearoylphosphatidylserin, Cholesterol.

Unter den Mischungen dieser Substanzen sind solche bevorzugt, die einen Anteil negativer Ladungsträger von nicht mehr als 20 mol% aufweisen. Weiter bevorzugt sind solche Mischungen, bei denen der Anteil negativer Ladungsträger zwischen 5 und 15 mol% liegt. Phosphatidylglycerole und Phosphatidylserine sind negative Ladungsträger in solchen Membranen.

Die erfindungsgemäßen Mischungen von Lipiden umfassen zwischen 35 und 45 mol% Cholesterol.

PH-sensitive Liposomen sind ebenfalls bekannt und umfassen insbesondere Cholesterolhemisuccinat (CHEMS), da dessen Carboxylgruppe durch unterhalb von pH 5 mehr und mehr entladen wird. Mit Vorteil wird dieses Lipid in Verbindung mit Phosphatidylethanolamin verwendet. Solche Liposomen mit einem Gehalt von 35 bis 50 mol% CHEMS und 65 bis 50 mol% Phosphatidylethanolamin sind bei neutralem pH-Wert stabil und zerfallen bei einem pH-Wert unterhalb von 5, wie er etwa bei der endosomalen Aufnahme erreicht wird. Anstelle des CHEMS kann auch ein anderes pH-sensitives Lipid eingesetzt werden, beispielweise Phosphatidylserin.

In einer ganz besonders bevorzugten Ausführung der Erfindung werden amphotere Liposomen verwendet. Ein besonderes Merkmal dieser Liposomen ist, dass deren Oberflächenladung sich mit dem pH-Wert der Umgebung ändert, von kationisch im sauren zu anionisch im basischen pH-Bereich.

Die WO 02/066012 beschreibt pH-sensitive amphotere Liposomen, deren amphoteres Verhalten durch die gleichzeitige Anwesenheit von kationischen und anionischen Ladungsträgern in der Membran bestimmt ist und sich durch geeignete Auswahl und Verhältnisse der Komponenten variieren lässt.

Als negativer Ladungsträger von amphoteren Liposomen nach WO 02/066012 werden bevorzugt natürliche Lipide eingesetzt, insbesondere: Dipalmitoylphosphatidylglycerol, Distearoylphosphatidylglycerol, Dipalmitoylphosphatidylserin, Distearoylphosphatidylserin, aber auch synthetisch hergestellte Lipide, wie beispielsweise Cholesterolhemisuccinat oder CHEMS, Diacylglycerolhemisuccinate.

Als kationischer Ladungsträger von amphoteren Liposomen nach WO 02/066012 können Lipide verwendet werden, bevorzugt:
DC-Chol 3-β-[N-(N',N'-dimethylethane) carbamoyl]cholesterol,
TC-Chol 3-β-[N-(N',N', N'-trimethylaminoethane) carbamoyl] cholesterol,
BGSC Bis-guanidinium-spermidine-cholesterol,
BGTC Bis-guanidinium-tren-cholesterol,
DOTAP (1,2-dioleoyloxypropyl)-N,N,N-trimethylammoniumchlorid,
DOSPER (1,3-dioleoyloxy-2-(6-Carboxy-spermyl)-propylamid),
DOTMA (1,2-dioleyloxypropyl)-N,N,N-trimethylammoniumchlorid) (Lipofectin®),
DORIE (1,2-dioleyloxypropyl)-3 dimethylhydroxyethyl ammoniumbromid),
DOSC (1,2-dioleoyl-3-succinyl-sn-glycerl cholinester), DOGSDSO (1,2-dioleoyl-sn-glycero-3-succinyl-2hydroxyethyl disulfide ornithin),
DDAB Dimethyldioctadecylammonium bromid,
DOGS ((C18)₂GlySper3⁺) N,N-dioctadecylamido-glycyl-spermin (Transfectam®),
(C18)₂Gly⁺ N,N-dioctadecylamido-glycin,
CTAB Cetyl-trimethylammoniumbromid,
CPyC Cetyl-pyridiniumchlorid,
DOEPC 1,2-dioleoyl-sn-glycero-3-ethylphosphocholin oder andere O-Alkyl-Phosphatidylcholin oder-ethanolamine und/oder
Amide aus Lysin, Arginin oder Ornithin und Phosphatidylethanolamin.

Auch pH-sensitiv positive Ladungsträger können Bestandteile von amphoteren Liposomen nach WO 02/066012 sein. Es werden bevorzugt Lipide verwendet, wie sie beispielsweise in der WO 02/066490 und WO 03/070220 offenbart sind, beispielsweise 4-(2-Aminoethyl)-Morpholino-Cholesterolhemisuccinat und/oder Histaminyl-Cholesterolhemisuccinat.

In einer bevorzugten Ausführung der erfinderischen Lehre wird zum Aufbau der amphoteren Liposomen eine Lipidmatrix mit neutralen Gerüstlipiden aufgebaut, die folgende Lipide miteinschließt:
Dimyrystoylphosphytidylcholin, Dipalmitoylphosphytidylcholin, Palmitoyloleoylphosphatidylcholin, Distearoylphosphytidylcholin, Cholesterol, sowie gereinigte Cholinfraktionen natürlichen Ursprungs wie Soja-Phosphatidylcholin oder Ei-Phosphatidylcholin.

Die WO 02/066489 beschreibt amphotere Liposomen, deren amphoteres Verhalten durch amphotere Ladungsträger in der Membran bestimmt wird, die sich vom Cholesterol ableiten, wie beispielsweise Nα-Histidinyl-Cholesterol-hemisuccinat. Die WO 03/070735 beschreibt Komponenten für weitere pH-sensitive Liposomen dieser Art.

Bevorzugt weisen die liposomalen Formulierungen vorteilhafte Mischungsverhältnisse auf. Unter den Mischungen dieser Substanzen sind solche bevorzugt, die einen Anteil negativer Ladungsträger von nicht mehr als 40 mol% aufweisen. Insbesondere bevorzugt sind solche Mischungen, bei denen der Anteil negativer Ladungsträger zwischen 5 bis 30 mol% liegt.

Unter den Mischungen dieser Substanzen sind solche bevorzugt, die einen Anteil positiver Ladungsträger von nicht mehr als 40 mol% aufweisen. Insbesondere bevorzugt sind solche Mischungen, bei denen der Anteil positiver Ladungsträger zwischen 5 und 30 mol% liegt.

Der molare Anteil an der Zusammensetzung der Liposomenmembran beträgt bei den Gerüstlipiden bevorzugt 30 bis 80 %, besonders bevorzugt 40 bis 70 %.

Die erfindungsgemäßen Mischungen von Lipiden umfassen zwischen 35 und 45 mol% Cholesterol

Werden amphotere Lipide zur Bildung der Liposomen eingesetzt, so beträgt der molare Anteil weniger als 40 mol%, bevorzugt 5 - 30 mol %.

Solche amphoteren Liposomen sind für die Ausführung der Erfindung besonders bevorzugt, da sie im Serum stabil sind und keine Aggregate bilden. Der Offenbarungsgehalt der zugehörigen Referenzen, der WO 02 066012, WO 02 066490, WO 02 066489, WO 03 070220, WO 03 070735 werden in die Offenbarung der Beschreibung aufgenommen.

Die Methoden zur Herstellung der wirkstoffhaltigen Liposomen sind dem Fachmann bekannt und können aus Standardwerken entnommen werden.

Mit besonderem Vorteil werden die Lipide in einem pharmakologisch akzeptablen Alkohol wie Ethanol, Isopropanol oder 1,2-Propandiol oder auch in DMSO gelöst und unter schnellem Mischen in die wässrige Lösung des Wirkstoffes verdünnt. Bei geeigneter Prozessführung, beispielsweise bei einer 10-fachen Verdünnung der organischen in die wässrige Phase, entstehen Liposomen im gewünschten Größenbereich zwischen 150 und 500 nm. Es ist dann keine weitere Veränderung der Liposomen durch Homogenisation oder Extrusion notwendig. Nicht eingeschlossener Wirkstoff kann abgetrennt werden.

Dazu eignen sich Verfahren wie Gelfiltration, Zentrifugation oder auch Dialyse, besonders Tangentialflussdialyse.

Die Erfindung betrifft auch pharmazeutische Mittel, die die erfindungsgemäßen liposomalen Formulierungen umfassen. Die erfindungsgemäßen Liposomen werden in einer pharmazeutischen Zubereitung formuliert. Diese umfasst die Verwendung und/oder Zumischung von Hilfsstoffen (Stabilisatoren, Antioxidantien), die eine gute Verträglichkeit, pharmazeutische Sicherheit und Herstellung und Lagerung unter GMP-Bedingungen sicherstellen. Dazu gehört beispielsweise die Verwendung von sterilen Salz-, Puffer- und/oder Zuckerlösungen.

Die erfindungsgemäßen Verbindungen werden in einer therapeutischen Menge mit einem Organismus in Kontakt gebracht; in solch einem Falle werden die erfindungsgemäßen Verbindungen als pharmazeutisches Mittel oder als Arzneimittel eingesetzt; demgemäß können diese Begriffe im Kontext der Erfindung synonym verwendet werden. Der Ausdruck therapeutische Menge bezeichnet wie hierin verwendet eine Menge, die Symptome einer Störung oder responsiven, pathologisch physiologischen Kondition verhindert oder verbessert. In bestimmten Ausführungsformen der vorliegenden Erfindung ist die verabreichte Menge ausreichend, eine Entzündung zu verhindern oder zu hemmen. Die Erfindung betrifft demgemäß - wie bereits ausgeführt - pharmazeutische Mittel oder Arzneimittel, die die erfindungsgemäßen Verbindungen umfassen, gegebenenfalls zusammen mit pharmazeutischen Hilfsstoffen.

Die an einem Gesunden im Falle der Prophylaxe bzw. an einem Patienten im Falle der Therapie zu verwendende Menge an erfindungsgemäßen Verbindungen wird formuliert und die Dosis gemäß üblicher medizinischer Praxis festgesetzt, wobei die zu behandelnde Störung, der Zustand des einzelnen Patienten, die Verabreichungsstelle, das Verabreichungsverfahren und andere den behandelnden Ärzten bekannte Faktoren berücksichtigt werden. In ähnlicher Weise hängt die Dosis der verabreichten erfindungsgemäßen Verbindungen von den Eigenschaften der Entzündung ab, von der in vivo Halbwertszeit erfindungsgemäßen Verbindungen im Plasma wie auch von der Konzentration der erfindungsgemäßen Verbindungen in der Formulierung, dem Verabreichungsweg, der Stelle und der Rate der Dosierung, der klinischen Toleranz des jeweiligen Individuums (Mensch und Tier), der pathologischen Affektion des Patienten und dergleichen, wie es Ärzten bzw. anderen Fachleuten bekannt ist. Es können während einer Abfolge aufeinander folgender Verabreichungen auch unterschiedliche Dosierungen eingesetzt werden.

Es ist zum Beispiel vorgesehen, dass Injektionen (intramuskulär oder subkutan oder in die Blutgefäße) ein Weg für die therapeutische Verabreichung der erfindungsgemäßen Verbindungen, beispielsweise der eingekapselten oder an Träger gebundenen erfindungsgemäßen Verbindungen, sind, obgleich die Zufuhr als Aerosol, über Katheter oder chirurgische Schläuche auch angewendet werden kann. Andere bevorzugte Wege umfassen im Handel erhältliche Vernebler für flüssige Formulierungen und Inhalationen von lyophilisierten oder aerolysierten Verbindungen und Suppositorien für rektale oder vaginale Verabreichung. Die Eignung der gewählten Parameter, zum Beispiel Dosis, Schema, Adjuvanzwahl und dergleichen, kann durch Entnahme von Serum-Aliquoten aus dem Patienten - das heißt dem Mensch oder dem Tier - und Testen im Verlauf der Applikationen bestimmt werden. Alternativ und begleitend dazu kann die Menge von T-Zellen oder anderen Zellen des Immunsystems auf herkömmliche Weise bestimmt werden, um einen Gesamtüberblick über die Entzündung des Patienten zu erhalten. Zusätzlich kann der klinische Zustand des Patienten auf die gewünschte Wirkung hin beobachtet werden. Da Entzündungen mit anderen Erkrankungen assoziiert sein können, ist es auch möglich, diese zusätzlich mit zu verfolgen.

Im Allgemeinen können sowohl wässrige Formulierungen als auch trockene erfindungsgemäße Verbindungen mit einem Exzipienten vermengt werden, um für eine stabilisierende Wirkung vor der Behandlung beispielsweise mit einem Lösungsmittel zu sorgen. Eine wässrige Lösung einer erfindungsgemäßen Verbindung kann eine erfindungsgemäße Verbindung in Suspension oder eine Lösung sein. Dem Fachmann sind weitere Zubereitungs-, Darreichungs- und Anwendungsformen bekannt, wie zum Beispiel als Gel, Emulsion, Aufgussformulierung, Tropfen, Konzentrat, Sirup, Boli, Aerosol, Spray und/oder Inhalat. Die Behandlung der Entzündungen umfasst Prophylaxe, Prävention, Diagnose, Verminderung, Therapie, Verlaufskontrolle und/oder Nachbehandlung.

Die erfindungsgemäße Verbindung kann in einer Lösung mit einem pharmazeutisch akzeptablen Konservierungsmittel eingebracht sein. Beispiele für geeignete Konservierungsmittel der Suspension bzw. Lösungen umfassen Phenol, Benzylalkohol, m-Kresol, Metylparaben, Propylparaben, Benzalkoniumchlorid und Benzethoniumchlorid in einer solchen Konzentration, dass die konservierende Wirkung eintritt, ohne dass die liposomale Formulierung nachteilig modifiziert wird. Im Allgemeinen können die Formulierungen der erfindungsgemäßen Verbindungen Komponenten in Mengen enthalten sein, die der Herstellung stabiler Formen nicht abträglich sind und in Mengen, die für wirksame, sichere pharmazeutische Verabreichungen geeignet sind. Beispielsweise können andere pharmazeutisch annehmbare, dem Fachkundigen bekannte Exzipienten einen Teil der erfindungsgemäßen Verbindungen oder Formulierungen bilden. Diese umfassen beispielsweise Salze, verschiedene Füller, zusätzliche Pufferagenzien, Chelatbildner, Antioxydanzien, Co-Lösungsmittel und dergleichen.

Dem Fachmann ist bekannt, dass künstliche bzw. natürliche Membranen von Liposomen eine immunstimulierende Wirkung haben können, insbesondere dann, wenn die Komponenten auf die Oberfläche der Liposomen gekoppelt werden oder im Inneren der Liposomen eingeschlossen sind oder einfach nur mit den Liposomen zusammen vermischt werden. Derartige Formulierungen von Liposomen können zum Beispiel zusammen mit den erfindungsgemäßen Liposomen parentral appliziert werden; selbstverständlich ist es auch möglich, die erfindungsgemäßen Liposomen so zu modifizieren, dass sie eine immunstimulierende Wirkung haben. Es ist möglich, derartige Formulierungen mit bekannten Methoden, wie zum Beispiel einem Spray, nasal auf die Schleimhäute zu applizieren. Eine mit dem Spray vorgenommene therapeutische Behandlung ist bevorzugt für die Behandlung von Entzündungen im Hals-Nasen-Ohren Bereich geeignet. Die liposomale Zusammensetzung, die zusammen mit der erfindungsgemäßen Zusammensetzung appliziert ist, kann einen oder mehrere zusätzliche pharmazeutische Träger umfassen, die ausgewählt sind aus oberflächenaktiven Stoffen und Absorptionsförderern wie zum Beispiel Gallensalzen und deren Derivaten, Fusidinsäure und deren Derivaten, Oleinsäure, Lecithin, Lysolecithinen usw., wasserabsorbierenden Polymeren wie zum Beispiel Glycofurol, Polyvinylpyrrolidon, Propylenglycol oder Polyacrylsäure, Gelatine, Cellulose und Derivaten usw.; Substanzen, welche den enzymatischen Abbau hemmen, wie zum Beispiel Aprotinin usw.; organischen Lösungsmitteln wie zum Beispiel Alkoholen, zum Beispiel Ethanol, Glycerol, Benzylalkohol usw.; oder Ethylacetat usw.; hydrophoben Mitteln wie zum Beispiel pflanzlichem Öl, Sojabohnenöl, Erdnussöl, Kokosnussöl, Maisöl, Olivenöl, Sonnenblumenöl, "Miglyolen" oder deren Mischungen usw.; pH-regulierenden Mitteln wie zum Beispiel Salpetersäure, Phosphorsäure, Essigsäure, Citraten usw.; Konservierungsmitteln und den osmotischen Druck regulierenden Mitteln wie zum Beispiel Glycerol, Natriumchlorid, Methylparaoxybenzoat, Benzoesäure usw.; Liposomen- und/oder Emulsionsformulierungen wie zum Beispiel Lecithinen usw.; mikroverkapselten Formulierungen; Treibmitteln wie zum Beispiel Butan.

Bei den Trägern, die Bestandteil von Arzneimitteln sein können - die die erfindungsgemäßen Verbindungen umfassen -, kann es sich im Sinne der Erfindung auch um Wasser-Öl-Emulsionen wie beispielsweise Montanide, Polylysin, Polyarginin-Verbindungen oder andere, wie zum Beispiel phosphat-gepufferte Kochsalzlösung, Wasser, sterile Lösungen und dergleichen mehr.

Das pharmazeutische Mittel im Sinne der Erfindung kann neben den Glucocorticoiden beispielsweise ein akzeptables Salz oder Komponenten dieser umfassen. Hierbei kann es sich beispielsweise um Salze anorganischer Säuren handeln wie zum Beispiel der Phosphorsäure bzw. um Salze organischer Säuren. Weiterhin ist es möglich, dass die Salze frei von Carboxylgruppen sind und von anorganischen Basen abgeleitet wurden wie zum Beispiel Natrium, Kalium, Ammonium, Calcium oder Eisenhydroxyde oder auch von organischen Basen wie Isopropylamin, Trimethylamin, 2-Ethylaminoethanol, Histidin und andere. Beispiele für flüssige Träger sind sterile wässrige Lösungen, die keine weiteren Materialien oder aktiven Ingredienzien umfassen und beispielsweise Wasser oder solche, die einen Puffer wie zum Beispiel Natriumphosphat mit einem physiologischen pH umfassen oder eine physiologische Salzlösung bzw. beides, wie zum Beispiel phosphatgepufferte Natriumchloridlösung. Weitere flüssige Träger können mehr als nur ein Puffersalz, wie zum Beispiel Natrium- und Kaliumchlorid, HEPES, Dextrose, Propylenglycol oder andere umfassen. Flüssige Zusammensetzungen der pharmazeutischen Mittel können zusätzlich eine flüssige Phase, jedoch unter dem Ausschluss von Wasser, umfassen. Beispiele solcher zusätzlichen flüssigen Phasen sind Glycerin, Pflanzenöle, organische Ester oder Wasser-Öl-Emulsionen. Die pharmazeutische Zusammensetzung bzw. das pharmazeutische Mittel enthält typischerweise einen Gehalt von mindestens 0,01 Gew% der erfindungsgemäßen Verbindungen bezogen auf die gesamte pharmazeutische Zusammensetzung.

Die jeweilige Dosis bzw. der Dosisbereich für die Gabe des erfindungsgemäßen pharmazeutischen Mittels ist groß genug, um den gewünschten prophylaktischen oder therapeutischen Effekt zu erreichen. Hierbei sollte die Dosis nicht so gewählt werden, dass unerwünschte Nebeneffekte dominieren. Im Allgemeinen wird die Dosis mit dem Alter, der Konstitution, dem Geschlecht des Patienten variieren sowie selbstverständlich auch in Bezug auf die Schwere der Erkrankung. Die individuelle Dosis kann sowohl in Bezug auf die primäre Erkrankung als auch in Bezug auf Eintreten zusätzlicher Komplikationen eingestellt werden. Die exakte Dosis ist durch einen Fachmann mit bekannten Mitteln und Methoden feststellbar, beispielsweise durch die Feststellung des Tumorwachstums in Abhängigkeit der Dosis bzw. in Abhängigkeit des Applikationsschemas oder der pharmazeutischen Träger und ähnlichem. Die Dosis kann hierbei je nach Patient individuell gewählt werden. Beispielsweise kann eine vom Patienten noch tolerierte Dosis des pharmazeutischen Mittels eine solche sein, deren Bereich im Plasma oder in einzelnen Organen lokal im Bereich von 0,1 bis 10000 µM liegt, bevorzugt zwischen 1 und 100 µM, wobei sich die Dosis auf das pharmazeutische Mittel, die liposomale Formulierung oder die Glucocorticoide beziehen kann. Alternativ kann die Dosis auch in Bezug auf das Körpergewicht des Patienten bezogen berechnet werden. Weiterhin ist es jedoch auch möglich, die Dosis nicht in Bezug auf den gesamten Patienten, sondern in Bezug auf einzelne Organe zu bestimmen. Dies wäre beispielsweise dann der Fall, wenn das erfindungsgemäße pharmazeutische Mittel beispielsweise in einem Biopolymer, eingebracht in den jeweiligen Patienten, in der Nähe bestimmter Organe mittels einer Operation platziert wird. Dem Fachmann sind mehrere Biopolymere bekannt, die Liposomen in einer gewünschten Art und Weise freisetzen können. In solch einem Fall wird das therapeutische Mittel als feste, gelartige oder als flüssige Komposition verabreicht.

In einer weiteren bevorzugten Ausführungsform der Erfindung sind die Träger ausgewählt aus der Gruppe umfassend Füllmittel, Streckmittel, Bindemittel, Feuchthaltemittel, Sprengmittel, Lösungsverzögerer, Resorptionsbeschleuniger, Adsorbtionsmittel, und/oder Gleitmittel, die so ausgewählt sind, dass sie die erfindungsgemäße liposomale Formulierung nicht beeinträchtigen.

Hierbei handelt es sich bei den Füll- und Streckmitteln bevorzugt um Stärken, Milchzucker, Rohrzucker, Glukose, Mannit und Kieselsäure, bei dem Bindemittel, bevorzugt Carbocymethylcellulose, Alginate, Gelatine, Polyvinylpyrrolidon, bei dem Feuchthaltemittel bevorzugt um Glycerin, bei dem Sprengmittel bevorzugt um Agar-Agar, Calciumcarbonat und Natriumcarbonat, bei dem Lösungsverzögerer vorzugsweise um Paraffin und bei dem Resorptionsbeschleuniger bevorzugt um quarternäre Ammoniumverbindungen, bei dem Adsorptionsmittel bevorzugt um Kaolin und Bentonit und bei dem Gleitmittel bevorzugt um Talkum, Calcium- und Magnesiumstearat oder Gemische der aufgeführten Stoffe.

In einer weiteren bevorzugten Ausführungsform der Erfindung werden die erfindungsgemäßen Verbindungen als Gel, Emulsion, Aufgussformulierung, Tropfen, Konzentrat, Sirup, Boli, Aerosol, Spray und/oder Inhalat zubereitet und/oder in dieser Form angewendet.

Der oder die Wirkstoffe - das heißt die erfindungsgemäßen Verbindungen - können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, zum Beispiel Polyethylenglycole, Fette, zum Beispiel Kakaofett und höhere Ester (zum Beispiel C₁₄-Alkohol mit C₁₆-Fettsäure) oder Gemische dieser Stoffe).

Salben, Pasten, Cremes und Gele können neben dem oder den Wirkstoffen die üblichen Trägerstoffe enthalten, zum Beispiel tierische und pflanzliche Fette, Wachse, Paraffine, Stärke, Tragant, Cellulosederivate, Polyethylenglycole, Silikone, Bentonite, Kieselsäure, Talkum und Zinkoxid oder Gemische dieser Stoffe.

Sprays können zusätzlich die üblichen Treibmittel, zum Beispiel Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, zum Beispiel Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydo-furfurylalkohol und Fettsäureester des Sorbitans oder Gemische dieser Stoffe enthalten. Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, zum Beispiel Wasser, Ethylalkohol, Propylenglykol, Suspendiermittel, zum Beispiel ethoxilierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die Arzneimittel können in Form einer sterilen injizierbaren Zubereitung, zum Beispiel als sterile injizierbare wässrige oder liposomale Suspension, vorliegen. Diese Suspension kann auch im Fachgebiet bekannten Verfahren unter Verwendung geeigneter Dispergier- oder Netzmittel und Suspendiermittel formuliert werden. Die sterile injizierbare Zubereitung kann auch eine sterile injizierbare Lösung oder Suspension in einem ungiftigen parenteral verträglichen Verdünnungs- oder Lösungsmittel, zum Beispiel als Lösung in 1,3-Butandiol, sein. Zu den verträglichen Vehikeln und Lösungsmitteln, die verwendet werden können, gehören Mannit, Wasser, Ringer-Lösung und isotonische Natriumchloridlösung.

Die genannten Formulierungsformen können auch Färbemittel, Konservierungsstoffe sowie geruchs- und geschmacksverbesserte Zusätze, zum Beispiel Süßmittel, zum Beispiel Saccharin, enthalten. Die erfindungsgemäßen Verbindungen sollen in den aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die aufgeführten pharmazeutischen Zubereitungen können außer den erfindungsgemäßen Verbindungen auch weitere pharmazeutische Wirkstoffe enthalten. Die Herstellung der oben aufgeführten pharmazeutischen Zubereitungen erfolgt in üblicher Weise nach bekannten Methoden, zum Beispiel durch Mischen des oder der Wirkstoffe mit dem oder den Trägerstoffen.

Die genannten Zubereitungen können bei Mensch und Tier entweder oral, nasal, rektal, regional - zum Beispiel in Gelenkbereichen oder ähnliches - parenteral (intravenös, intramuskulär, subkutan), intracisternal, intravaginal, intraperitoneal, lokal, dermal (Puder, Salbe, Tropfen) und zur Therapie von Entzündungen in Hohlräumen, Körperhöhlen angewendet werden. Als geeignete Zubereitung kommen Injektionslösungen, Lösungen und Suspensionen für die orale Therapie, Gele, Aufgussformulierungen, Emulsionen, Salben oder Tropfen in Frage. Zur lokalen Therapie können ophtalmologische und dermatologische Formulierungen oder Lösungen verwendet werden. Bei Tieren kann die Aufnahme auch über das Futter oder Trinkwasser in geeigneten Formulierungen erfolgen. Ferner können Gele, Tabletten, Retard-Tabletten, Premixe, Konzentrate, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Die Wirkstoffmenge, das heißt die Menge einer erfindungsgemäßen Verbindung, die mit den Trägermaterialien kombiniert wird, um eine einzige Dosierungsform zu erzeugen, wird von dem Fachmann in Abhängigkeit von dem zu behandelnden Wirt, von der zu behandelnden Entzündung und der besonderen Verabreichungsart variieren können. Nach Besserung des Zustandes eines Wirts bzw. eines Patienten kann der Anteil der wirksamen Verbindung in der Zubereitung so geändert werden, dass eine Erhaltungsdosis vorlegt. Anschließend kann die Dosis oder Frequenz der Verabreichung oder beides als Funktion der Symptome auf eine Höhe verringert werden, bei der der verbesserte Zustand beibehalten wird. Wenn die Symptome auf das gewünschte Niveau gelindert worden sind, sollte die Behandlung aufhören. Patienten können jedoch eine Behandlung mit Unterbrechung auf Langzeitbasis nach beliebigem Wiederauftreten von Erkrankungssymptomen benötigen. Demgemäß ist der Anteil der Verbindungen, das heißt ihre Konzentration, in der Gesamtmischung der pharmazeutischen Zubereitung ebenso wie ihre Zusammensetzung oder Kombination variabel und kann vom Fachmann aufgrund seines Fachwissens modifiziert und angepasst werden.

Dem Fachmann ist bekannt, dass die erfindungsgemäßen Verbindungen mit einem Organismus, bevorzugt einem Menschen oder einem Tier, auf verschiedenen Wegen in Kontakt gebracht werden können. Weiterhin ist dem Fachmann bekannt, dass insbesondere die pharmazeutischen Mittel in verschiedenen Dosierungen appliziert werden können. Die Applikation sollte hierbei so erfolgen, dass die Entzündung möglichst effektiv bekämpft wird bzw. der Ausbruch einer solchen Krankheit in einer prophylaktischen Gabe verhindert wird. Die Konzentration und die Art der Applikation kann vom Fachmann durch Routineversuche eruiert werden. Bevorzugte Applikationen der erfindungsgemäßen Verbindungen sind die orale Applikation in Form von Saft, Tropfen, Kapseln oder ähnlichem, die rektale Applikation in Form von Zäpfchen, Lösungen und ähnlichem, parenteral in Form von Injektionen, Infusionen und Lösungen, Inhalation von Dämpfen und Aerosolen sowie lokal in Form von Salben, Pflastern, Umschlägen, Spülungen und ähnlichem. Bevorzugt erfolgt das In-Kontakt-Bringen der erfindungsgemäßen Verbindungen prophylaktisch oder therapeutisch. Bei einer prophylaktischen Gabe soll die Ausbildung der Entzündung zumindest dergestalt verhindert werden, dass eine weitere Entzündung stark vermindert wird oder nahezu vollständig eliminiert wird. Bei einem therapeutischen In-Kontakt-Bringen liegt bereits eine manifeste Entzündung des Patienten vor und die bereits im Körper vorhandenen Entzündungsherde sollen gehemmt werden. Weitere hierfür bevorzugte Applikationsformen sind beispielsweise die subkutane, die sublinguale, die intravenöse, die intramuskuläre, die intraperitoneale und/oder die topische.

Neben den bereits ausgeführten Konzentrationen bei der Anwendung der erfindungsgemäßen Verbindungen können in einer bevorzugten Ausführungsform die Verbindungen weiterhin in einer Gesamtmenge von 0,005 bis 500 mg/kg Körpergewicht je 24 Stunden eingesetzt werden, bevorzugt von 0.05 bis 100 mg/kg Körpergewicht. Hierbei handelt es sich vorteilhafterweise um eine therapeutische Menge, die verwendet wird, um die Symptome einer Störung oder responsiven, pathologisch physiologischen Kondition zu verhindern oder zu verbessern. Die verabreichte Menge ist ausreichend, um die Entzündung zu hemmen.

Selbstverständlich wird die Dosis vom Alter, der Gesundheit und dem Gewicht des Empfängers, dem Grad der Krankheit, der Art einer notwendigen gleichzeitigen Behandlung, der Häufigkeit der Behandlung und der Art der gewünschten Wirkungen und der Nebenwirkungen abhängen. Die tägliche Dosis von 0,005 bis 500 mg/kg Körpergewicht kann einmalig oder mehrfach angewendet werden, um die gewünschten Ergebnisse zu erhalten. Die Dosishöhen pro Tag sind bei der Vorbeugung und bei der Behandlung anwendbar. Typischerweise werden insbesondere pharmazeutischen Mittel zur etwa 1- bis 10-maligen Verabreichung pro Tag oder alternativ oder zusätzlich als kontinuierliche Infusion verwendet. Sie können aber auch in Abständen von 2 - 10 Tagen verabreicht werden. Solche Verabreichungen können als chronische oder akute Therapie angewendet werden. Die Wirkstoffmengen, die mit den Trägermaterialien kombiniert werden, um eine einzelne Dosierungsform zu erzeugen, können in Abhängigkeit von dem zu behandelnden Wirt und der besonderen Verabreichungsart selbstverständlich variieren. Bevorzugt ist es, die Targetsdosis auf 2 bis 5 Applikationen zu verteilen, wobei bei jeder Applikation 1 bis 2 Tabletten, Kapseln, Lösungen mit einem Wirkstoffgehalt von 0,05 bis 500 mg/kg Körpergewicht verabreicht werden. Selbstverständlich ist es möglich, den Wirkstoffgehalt auch höher zu wählen, beispielsweise bis zu einer Konzentration bis 5000 mg/kg. Beispielsweise können Kapseln, die die erfindungsgemäßen Liposomen umfassen, auch retardiert sein, wodurch sich die Anzahl der Applikationen pro Tag auf 1 bis 3 vermindert oder es können mehrere Tage dazwischen liegen. Der Wirkstoffgehalt der retardierten Kapseln kann 3 bis 3000 mg betragen.

Wenn der Wirkstoff - wie ausgeführt - durch eine Injektion verabreicht wird, ist es bevorzugt, 1- bis 10-mal pro Tag bzw. durch Dauerinfusion den Wirt mit den erfindungsgemäßen Verbindungen in Kontakt zu bringen, wobei Mengen von 0,04 bis 4000 mg pro Tag bevorzugt sind. Die bevorzugten Gesamtmengen pro Tag haben sich in der Humanmedizin und in der Veterinärmedizin als vorteilhaft erwiesen. Es kann erforderlich sein, von den genannten Dosierungen abzuweichen und zwar in Abhängigkeit von der Art und dem Körpergewicht des zu behandelnden Wirts, der Art und der Schwere der Erkrankung, der Art der Zubereitung der Applikation des Arzneimittels sowie dem Zeitraum bzw. dem Intervall, innerhalb welchem die Verabreichung erfolgt. So kann es in einigen Fällen bevorzugt sein, den Organismus mit weniger als den genannten Mengen in Kontakt zu bringen, während in anderen Fällen die angegebene Wirkstoffmenge überschritten werden muss. Die Festlegung der jeweils erforderlichen optimalen Dosierungen und der Applikationsart der Wirkstoffe kann durch den Fachmann aufgrund seines Fachwissens leicht erfolgen.

In einer weiteren besonders bevorzugten Ausführungsform der Erfindung werden die erfindungsgemäßen Verbindungen in einer Einzelgabe von 0,01 bis 80, insbesondere von 0,3 bis 40 mg/kg Körpergewicht eingesetzt. Wie auch die Gesamtmenge pro Tag kann auch die Menge der Einzelgabe pro Applikation von dem Fachmann aufgrund seines Fachwissens variiert werden. Die erfindungsgemäß verwendeten Verbindungen können in den genannten Einzelkonzentrationen und Zubereitungen zusammen mit dem Futter bzw. mit Futterzubereitungen oder mit dem Trinkwasser auch in der Veterinärmedizin gegeben werden.

In einer weiteren bevorzugten Ausführungsform der Erfindung können die erfindungsgemäßen Verbindungen mit mindestens einem anderen bekannten pharmazeutischen Mittel eingesetzt werden. Das heißt, die erfindungsgemäßen Verbindungen können in einer prophylaktischen oder therapeutischen Kombination in Verbindung mit den bekannten Arzneimitteln eingesetzt werden. Diese Kombinationen können gemeinsam, zum Beispiel in einer einheitlichen pharmazeutischen Formulierung verabreicht werden, oder getrennt, zum Beispiel in Form einer Kombination aus Tabletten, Injektion oder anderen Medikamenten, die zu gleichen oder zu unterschiedlichen Zeiten verabreicht werden, mit dem Ziel, die gewünschte prophylaktische oder therapeutische Wirkung zu erzielen. Bei diesen bekannten Mitteln kann es sich um Mittel handeln, die die Wirkung der erfindungsgemäßen Verbindungen steigern.

Selbstverständlich ist es auch möglich, die erfindungsgemäßen Verbindungen, insbesondere die pharmazeutischen Mittel, allein oder zusammen mit anderen Mitteln in einer Therapie, beispielsweise in einer Kombinationstherapie, als regionale Therapie einzusetzen.

Selbstverständlich können die erfindungsgemäßen Verbindungen in Kombination mit anderen bekannten Anti-Entzündungsmitteln verwendet werden. Dem Fachmann sind derartige Mittel bekannt. Die erfindungsgemäßen Verbindungen können demgemäß mit allen herkömmlichen Mitteln, insbesondere anderen Arzneimitteln, verabreicht werden, die für die Verwendung verfügbar sind, entweder als einzelne Arzneimittel oder in Kombination von Arzneimitteln. Sie können allein verabreicht werden oder in Kombination mit diesen.

Die pharmazeutische Zusammensetzung kann in Substanz oder als wässrige Lösung vorliegen zusammen mit anderen Materialen wie Konservierungsmitteln, Puffersubstanzen, Mitteln die zur Einstellung der Osmolarität der Lösung vorgesehen sind etc.

Die Erfindung betrifft auch einen Kit und dessen Verwendung in der Medizin. Es ist bevorzugt, die erfindungsgemäßen Verbindungen oder den Kit, der diese umfasst, in einer Kombinationstherapie, insbesondere zur Behandlung von Entzündungen, zu verwenden.

Die erfindungsgemäßen Liposomen finden Anwendung bei der systemischen Behandlung von inflammatorischen und Autoimmun-Erkrankungen mit Cortikoiden.

Bevorzugte Indikationen sind die Autoimmunerkrankungen. Dazu gehören unter anderem die systemischen Vaskulitiden, Lupus erythematodes, Polymyositis, Polymyalgia rheumatica, rheumatisches Fieber, rheumatische rheumatoide Arthritis, systemische Sklerose, reaktive Arthritiden, Neurodermitis, Psoriasis, Morbus Crohn, Colitis ulcerosa, Multiple Sklerose, Asthma bronchiale und andere mehr.

Die erfindungsgemäßen Liposomen, pharmazeutischen Mittel und/oder der Kit können zur Prophylaxe oder Behandlung von pathogenen Modifikationen eingesetzt werden, bevorzugt von (i) Entzündungen, besonders bevorzugt von (ii) Autoimmunerkrankungen, ganz besonders bevorzugt von (iii) Arthritis.
(i) Entzündungen im Sinne der Erfindung sind die vom Bindegewebe und den Blutgefäßen getragene Reaktion des Organismus auf einen äußeren oder innerlich ausgelösten Entzündungsreiz mit dem Zweck, diesen zu beseitigen oder zu inaktivieren und die reizbedingte Gewebsschädigung zu reparieren. Auslösend wirken mechanische Reize (Fremdkörper, Druck, Verletzung) und andere physikalische Faktoren (ionisierende Strahlen, UV-Licht, Wärme, Kälte), chemische Stoffe (Laugen, Säuren, Schwermetalle, bakterielle Toxine, Allergene und Immunkomplexe) sowie Erreger(Mikroorganismen, Würmer, Insekten) bzw. krankhafte Stoffwechselprodukte, entgleiste Enzyme, bösartige Tumoren. Das Geschehen beginnt mit einer kurzen Arteriolenverengung (durch Adrenalinwirkung) mit Mangeldurchblutung und Gewebsalteration, gefolgt von der Entwicklung der klassischen örtlichen Entzündungszeichen (Kardinalsymptome; nach GALEN und CELSUS), das heißt von Rötung (= Rubor; Gefäßerweiterung durch Histamin), Wärme (= Calor; durch örtliche Stoffwechselsteigerung), Schwellung (= Tumor; durch Austritt eiweißreicher Flüssigkeit aus den - unter anderem durch Histamin - veränderten Gefäßwänden, unterstützt durch die verlangsamte Blutzirkulation im Sinne der Prästase bis Stase), Schmerz (= Dolor; als Folge der erhöhten Gewebsspannung und schmerzauslösender Entzündungsprodukte, zum Beispiel Bradykinin) und Funktionsstörung (= Functio laesa). Der Vorgang wird ergänzt durch Störung des Elektrolythaushaltes (Transmineralisation), Einwanderung neutrophiler Granulozyten und Monozyten durch die Gefäßwände (s.a. Leukotaxis), letzteres mit dem Zweck, den Entzündungsreiz und geschädigte bis neukrotische Zellen zu beseitigen (Phagozytose); ferner wandern Lymphozyten-Effektorzellen ein, die zur Bildung spezifischer Antikörper gegen den Entzündungsreiz führen (Immunreaktion), sowie Eosinophile (in der Heilungsphase bzw. - sehr frühzeitig - bei allergisch-hyperergischem Geschehen). Durch die bei der Reaktion erfolgende Aktivierung des Komplementsystems werden Bruchstücke (C3a und C5a) dieses Systems frei, die - wie das Histamin und Bradykinin - als Mediatoren der Entzündung wirken, und zwar im Sinne der Anregung der Chemotaxis der zitierten Blutzellen; ferner wird die Blutgerinnung aktiviert. In der Folge tritt eine Schädigung (Dystrophie und Koagulationsnekrose) des zugeordneten Organparenchyms ein. Der Gesamtorganismus reagiert je nach Intensität und Art der Entzündung mit Fieber, Stress (s.a. Adaptationssyndrom), Leukozytose und Veränderungen in der Zusammensetzung der Plasmaproteine (Akute-Phase-Reaktion), die zu einer beschleunigten Blutkörperchensenkungsreaktion führen. Bevorzugte Entzündungen im Sinne der Erfindung sind die eitrige, die exudative, die fibrinöse, die gangräneszierende, die granulomatöse, die hämorrhagische, die katarrhalische, die nekrotisierende, die proliferative oder produktive, die pseudomembranöse, die seröse, die spezifische und/oder die ulzeröse Entzündungen.
(ii) Autoimmunerkrankungen im Sinne der Erfindung sind Krankheiten, die ganz oder teilweise auf die Bildung von Autoantikörpern und deren schädigende Einwirkung auf den Gesamtorganismus bzw. Organsysteme, das heißt auf Autoaggression zurückzuführen sind. Eine Klassifikation ist als organspezifische, intermediäre und/oder systemische Autoimmunerkrankung möglich. Bevorzugte organspezifische Autoimmunerkrankungen sind HASHIMOTO Thyreoiditis, primäres Myxödem, Thyreotoxikose (BASEDOW Krankheit), perniziöse Anämie, ADDISON Krankheit, Myasthenia gravis und/oder juveniler Diabetes mellitus. Bevorzugte intermediäre Autoimmunkrankheiten sind GOODPASTURE Syndrom, autoimmune hämolytische Anämie, autoimmune Leukopenie, idiopathische Thrombozytopenie, Pemphigus vulgaris, sympathische Ophthalmie, primäre biliäre Zirrhose, Autoimmunhepatitis, Colitis ulcerosa und/oder SJÖGREN Syndrom. Bevorzugte systemische Autoimmunkrankheiten sind rheumatoide Arthritis, rheumatisches Fieber, systemischer Lupus erythematodes, Dermatomyositis/Polymyositis, progressive systemische Sklerose, WEGENER Granulomatose, Panarteriitis nodosa und/oder Hypersensitivitätsangiitis. Typische Autoimmunkrankheiten sind Thyreotoxikose, Schilddrüsen-bedingtes Myxödem, HASHIMOTO Thyreoiditis, generalisierte Endokrinopathie, perniziöse Anämie, chronische Gastritis Typ A, Krankheiten einzelner oder aller korpuskulären Elemente des Blutes (zum Beiapiel autoimmunhämolytische Anämie, idiopath. Thrombozytopenie bzw. -pathie; idiopath. Leukopenie bzw. Agranulozytose), Pemphigus vulgaris und Pemphigoid, sympathische Ophthalmie und manche Uveitis-Formen, primär biliäre Leberzirrhose und chronisch aggressive Autoimmunhepatitis, Diabetes mellitus Typ I, CROHN Krankheit und Colitis ulcerosa, SJÖGREN Syndrom, ADDISON Krankheit, Lupus erythematodes disseminatus und als diskoide Form dieser Krankheit, als Dermatomyositis und Sklerodermie, rheumatoide Arthritis (= primär-chronische Polyarthritis), Antiglomerulusbasalmembran-Nephritis. Grundlage sind eine aggressive Immunreaktion infolge Zusammenbruchs der Immuntoleranz gegenüber Selbst-Derterminanten und eine Abnahme der Aktivität der T-Suppressorzellen (mit Lymphozytenmarker T 8) bzw. ein Übergewicht der T-Helferzellen (mit Lymphozytenmarker T 4) über die Suppressorzellen; ferner ist die Bildung von Autoantigenen möglich, zum Beispiel durch Verbindung von Wirtsproteinen mit Haptenen (zum Beispiel Arzneimittel), durch ontogenetisches Gewebe, das sich erst nach Entwicklung der Selbsttoleranz entwickelt und für durch Änderungen der Konformation der Proteine demaskierte Proteinkomponenten im Zusammenhang zum Beispiel mit Infektion durch Viren oder Bakterien; ferner für im Zusammenhang mit Neoplasien entstandene neue Proteine.
(iii) Arthritis im Sinne der Erfindung sind Gelenkentzündungen als Oberbegriff für primäre bzw. sekundäre entzündliche Gelenkerkrankungen. Bevorzugte Arthritiserkrankungen sind die juvenile chronische, die A. mutilans, die paraneoplastische, die A. psoriatica, die reaktive und/oder die rheumatoide Arthritis.

Auch zur Unterdrückung der Abstoßung von Transplantaten oder bei der Behandlung des Typ-I-Diabetes können liposomale Formulierungen von Glucocorticoiden mit Vorteil verwendet werden.

Eine ganz besonders bevorzugte Indikation ist die rheumathische Arthritis.

So wird bei gleicher Wirkstoffmenge ein erheblich besserer therapeutischer Effekt beobachtet. Eine antigeninduzierte Arthritis in Ratten konnte mit 0,375 mg liposomalem Dexamethasonphosphat pro kg Körpergewicht nahezu vollständig unterdrückt werden. Tiere, die eine identische Dosis des unverpackten Wirkstoffs erhielten, entwickelten nach kurzer Besserung alle Symptome des Tiermodells.

Im Folgenden soll die Ausführung der erfinderischen Lehre an einigen Beispielen verdeutlicht werden, ohne dass sie auf diese Beispiele beschränkt ist.

### Beispiel 1

### Herstellung Dexamethason-Phosphat gefüllter Liposomen

Ein Gemisch aus 50 mol% DPPC, 10 mol% DPPG und 40 mol% Chol wird in Chloroform gelöst und anschließend im Rotationsverdampfer im Vakuum vollständig getrocknet.

Der Lipidfilm wird mit so viel Dexamethason-Phosphat-Lösung (25 mg/ml Dexamethason-Phosphat in 10 mM Hepes, 150 mM NaCl pH 7.5) versetzt, dass eine 100 mM Suspension entsteht. Anschließend wird diese Suspension für 45 Minuten im Wasserbad bei 50 °C unter Rotieren hydratisiert und für weitere 5 Minuten im Ultraschallbad behandelt. Danach wird die Lösung eingefroren.

Nach dem Auftauen werden die Liposomen mehrfach durch eine Membran mit einer Porenweite von 400 nm extrudiert. Die Abtrennung des nicht eingeschlossenen Dexamethason-Phosphats erfolgt über Gelfiltration.

### Beispiel 2

### Bestimmung der Freisetzung von Dexamethason-Phosphat

Liposomen wurden wie in Beispiel 1 hergestellt und mit Puffer (10 mM Hepes, 150 mM NaCl pH 7.5)auf eine Konzentration von 12 mM Lipid verdünnt. Anschließend werden sie bei 37 °C inkubiert. Aliquots dieser Inkubationslösung werden zu definierten Zeitpunkten (vergleiche nachfolgende Tabelle) entnommen. Die entnommenen Aliquots werden mittels RP-HPLC auf ihren Gehalt an Dexamethason-Phosphat vermessen.

**Tabelle 1**

| Formulierung | Lipid | mol % | 0 Stunden | 24 Stunden | 120 Stunden |
|---|---|---|---|---|---|
| A | DPPC/DPPG/Chol | 50:10:40 | 1, 1 | 6,0 | 7,3 |

Freisetzung von Dexamethason-Phosphat zu unterschiedlichen Zeitpunkten nach Inkubation bei 37 °C in Puffer in % der eingeschlossenen Menge

Die Liposomen zeigen über den untersuchten Messzeitraum eine hinreichende Stabilität. Über einen Zeitraum von 5 Tagen werden weniger als 8 % des eingeschlossenen Wirkstoffs freigesetzt.

### Beispiel 3

### Einsatz von liposomalem Dexamethason-Phosphat

In den Versuchstieren (Ratten) wurde entsprechend Buchner ("Behandlung der antigeninduzierten Arthritis der Ratte mit Anti-Makrophagenprinzipien und monoklonalen Anti-CD4 Antikörpern", Dissertation 1996 an der Friedrich-Alexander-Universität Erlangen-Nürnberg, S. 129) eine antigeninduzierte Arthritis ausgelöst.

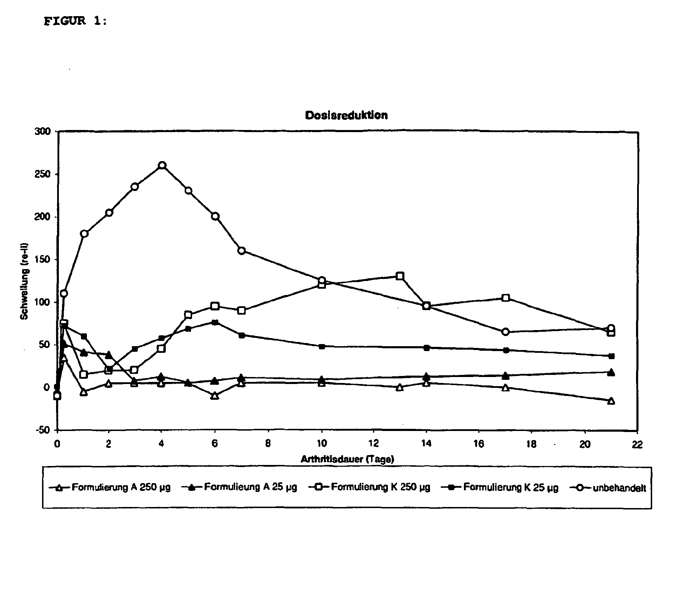

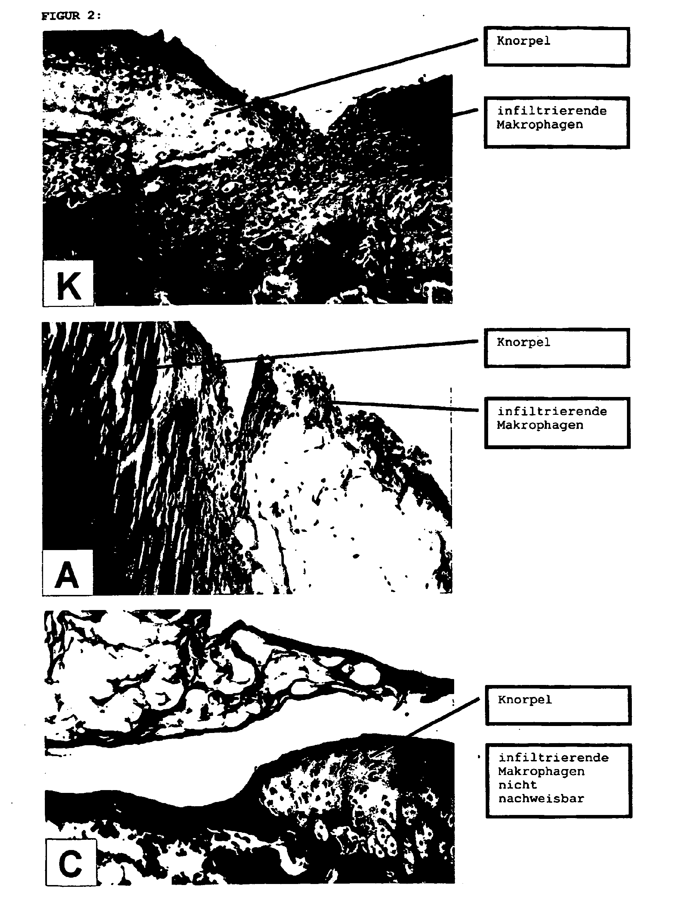

Die Arthritis wurde am Tag 0 entsprechend Buchner durch intraartikuläre Injektion des Antigens in den Synovialspalt im rechten Knie ausgelöst. Die arthritischen Tiere wurden 6, 24 und 48 Stunden nach Induktion der Arthritis intravenös mit liposomalem Dexamethason-Phosphat (Formulierung A 3,75 mg/kg) behandelt. Als Kontrolle dienen freies Dexamethason-Phosphat (Formulierung K, ebenfalls 3,75 mg/kg) und physiologische Salzlösung (Saline). Die Wirkung der Probengabe wurde anhand folgender Parameter ermittelt:
- Bestimmung der Gelenkschwellung (vgl. Figur 1)
- histologische Begutachtung von Entzündungsparametern und Parametern der Gelenkknorpelzerstörung (vgl. Figur 2 und Tabelle 2)

**Tabelle 2**

| Formulierung | Grad der Knorpelzerstörung | Grad der Gelenkentzündung |
|---|---|---|
| Saline | 4,0 | 5,5 |
| Formulierung K | 2,75 | 3,5 |
| Formulierung A | 0,25 | 0,25 |

Grad der Gelenkknorpelzerstörung und Gelenkentzündung der arthritischen Tiere nach Behandlung
0 - keine Veränderungen im Vergleich zum gesunden Knie
6 - starke Veränderungen im Vergleich zum gesunden Knie

Sowohl Gelenkknorpelzerstörung als auch Entzündungsreaktionen werden durch Behandlung mit liposomalem Dexamethason-Phosphat wirksam verringert. Freier Wirkstoff in der angegebenen Konzentration ist erheblich schlechter wirksam.

### Beispiel 4

### Einsatz von liposomalem Dexamethason-Phosphat zur Dosisreduktion

Eine Arthrits wurde analog Beispiel 3 an Ratten ausgelöst und diese mit folgenden Formulierungen behandelt, die nur ein Zehntel der in Beispiel 3 angegebenen Dosis Dexamethason-Phosphat enthielten:
Formulierung. A 25 µg = 0,375 mg/ kG KG
Formulierung K 25 µg = 0,375 mg/ kG KG

Die gemessenen Gelenkschwellungen werden auch in der Figur 1 dargestellt. Ein fast gleicher therapeutischer Effekt ist auch mit der verringerten Dosis festzustellen, die Gelenkschwellung bildet sich bei Formulierung A nach etwa 3 Tagen vollständig zurück, was bei Formulierung K nicht auftritt.

### Beispiel 5

### Visualiserung der Liposomeverteilung im Körper

Als Label wurde der Fluoreszenzfarbstoff Cy5.5^{™} (Amersham Bioscience) eingesetzt, der ein Anregungsmaximum bei 675 nm und ein Fluoreszenzmaximum bei 694 nm, das heißt im. nahen Infrarot hat und durch Bestrahlung durch die Haut angeregt werden kann. Cy5.5^{™}-BSA wurde durch Kopplung des Cy5.5^{™}-NHS-Esters an BSA nach dem Protokoll von Amersham-Bioscience erhalten.

Mit Cy5.5^{™}-BSA gefüllte Liposomen wurden in der Zusammensetzung DPPC/DPPG/Chol 50:10:40 analog Beispiel 1 hergestellt.
- nicht eingeschlossenes Cy5.5^{™} wurde durch Waschen (Hepes 10mM, mit 150 mM NaCl) und Sedimentation (65000 rpm mit Beckman Rotor 100.4, 40 min, bei 21°C) abgetrennt und die Liposomen mit physiologischer Kochsalzlösung auf 6,5 mM im Lipid verdünnt (Gehalt an Cy5.5^{™}: 8 nmol/mL).
- Versuchstiere: Mäuse mit etablierter AIA (antigen-induzierter Arthritis), analog wie im vorherigen Beispiel beschrieben.
- Am 7. Tag nach Auslösung der Arthritis wurde den Tieren intravenös in die Schwanzvene einmal 100 µL liposomal verkapseltes Cy5.5^{™} appliziert.
- die Fluoreszenzmessung erfolgte nach 6 Stunden mit NIR-Fotografie (Nah-Infarot), dabei wurden arthritisches und gesundes Kniegelenk getrennt vermessen.

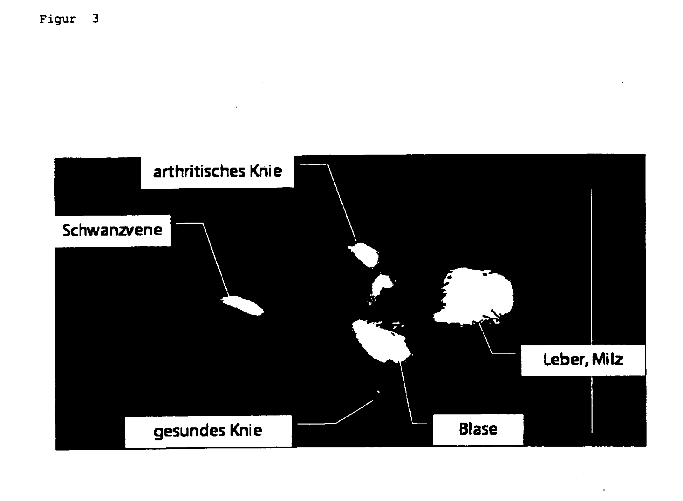

Figur 3 zeigt als helle Stellen, Orte, an denen eine Anreicherung des Farbstoffes Cy5.5^{™} stattgefunden hat. Die Signalstärken sind in etwa wie folgt:
900 im arthritischen Gelenk;
600 im gesunden Gelenk;
2500 in Leber und Milz.

Durch Messung der Zunahme des Fluoreszenzsignals konnte die Aufnahme der Liposomen in die Gelenke in einer Zeitreihe verfolgt werden.

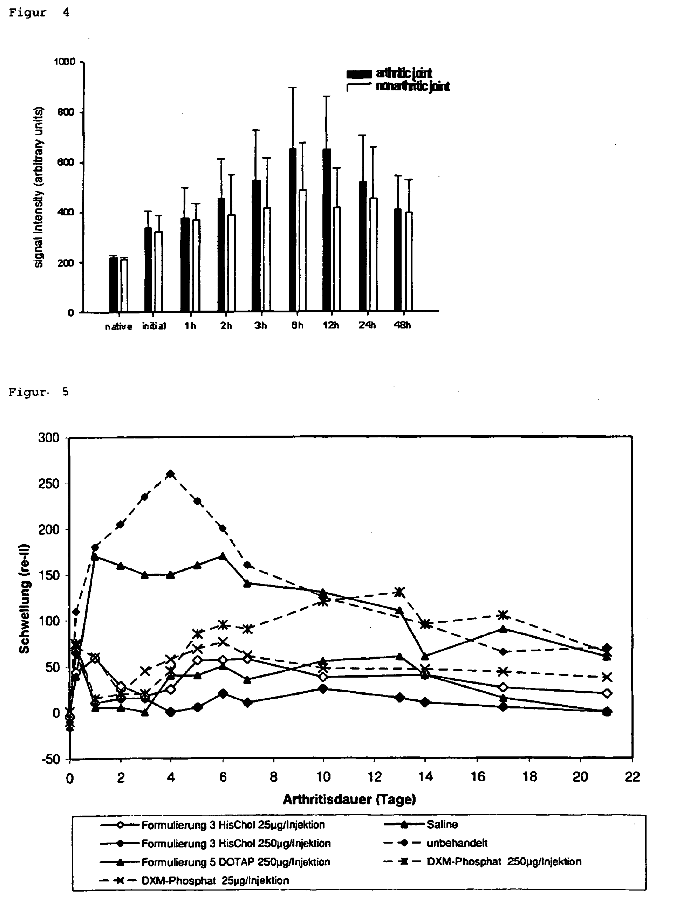

Dies wird in Figur 4 gezeigt. Man erkennt, dass im arthritischen Gelenk für den Zeitraum der ersten 12 bis 24 Stunden eine stärkere Anreicherung des Cy5.5^{™} erfolgt als im gesunden Gelenk.

### Beispiel 6

### Herstellung Dexamethason-Phosphat gefüllter amphoterer Liposomen

Ein Gemisch aus 60 mol% POPC, 20 mol% MoChol und 20 mol% Chems wird in Chloroform gelöst und anschließend im Rotationsverdampfer im Vakuum vollständig getrocknet.

Der Lipidfilm wird mit so viel Dexamethason-Phosphat-Lösung (25 mg/ml Dexamethason-Phosphat in 10 mM Hepes, 150 mM NaCl pH 7.5) versetzt, dass eine 100 mM Suspension entsteht. Anschließend wird diese Suspension für 45 Minuten im Wasserbad bei 50 °C unter Rotieren hydratisiert und für weitere 5 Minuten im Ultraschallbad behandelt. Danach wird die Lösung eingefroren.

Nach dem Auftauen werden die Liposomen mehrfach durch eine Membran mit einer Porenweite von 400 nm extrudiert. Die Abtrennung des nicht eingeschlossenen Dexamethason-Phosphates erfolgt über Gelfiltration.

Es werden analog folgende mit Dexamethason-Phosphat gefüllten Liposomen hergestellt:

**Tabelle 3**

| Lipid | mol% |
|---|---|
| POPC/MoChol/Chems | 60:20:20 |
| POPC/HisChol/Chems | 60:20:20 |
| POPC/DOTAP/Chems | 60:10:30 |
| POPC/HistChol/Chol | 60:20:20 |
| DPPC/AC/Chol | 50:10:40 |

Beispiele möglicher Liposomenformulierungen für den Einschluss von wasserlöslichen Glucocorticoiden.

### Beispiel 7

### Bestimmung der Freisetzung von Dexamethason-Phosphat aus amphoteren Liposomen

Liposomen wurden wie in Beispiel 6 hergestellt und mit Puffer (10 mM Hepes, 150 mM NaCl pH 7.5)auf eine Konzentration von 12 mM Lipid verdünnt. Anschließend werden sie bei 37 °C inkubiert. Aliquots dieser Inkubationslösung werden zu definierten Zeitpunkten (vgl. nachfolgende Tabelle) entnommen. Die entnommenen Aliquots werden mittels RP-HPLC auf ihren Gehalt an Dexamethason-Phosphat vermessen.

**Tabelle 4**

| Formulierung | Lipid | mol% | 0 Stunden | 24 Stunden | 120 Stunden |
|---|---|---|---|---|---|
| | POPC/MoChol/Chems | 60:20:20 | 4,6 | 10,5 | 10,0 |
| B | POPC/HisChol/Chems | 60:20:20 | 6,7 | 14,1 | 10,8 |
| C | POPC/DOTAP/Chems | 60:10:30 | 4,4 | 4,2 | - |
| | POPC/HistChol/Chol | 60:20:20 | 8,7 | 7,0 | - |
| | DPPC/AC/Chol | 50:10:40 | 1,7 | 7,8 | 7,4 |

Freisetzung von Dexamethason-Phosphat zu unterschiedlichen Zeitpunkten nach Inkubation bei 37 °C in Puffer in % der eingeschlossenen Menge

Die unterschiedlichen Liposomen zeigen über den untersuchten Messzeitraum eine hinreichende Stabilität. Über einen Zeitraum von 5 Tagen werden weniger als 6 %, oft weniger als 4 % des eingeschlossenen Wirkstoffs freigesetzt.

### Beispiel 8

### Einsatz von Dexamethason-Phosphat formuliert in amphoteren Liposomen

In den Versuchstieren wurde analog Beispiel 3 eine antigeninduzierte Arthritis ausgelöst. Die Arthritis wurde am Tag 0 durch intraartikuläre Injektion des Antigens in den Synovialspalt im rechten Knie ausgelöst. Die arthritischen Tiere wurden 6, 24 und 48 Stunden nach Induktion der Arthritis intravenös mit liposomalem Dexamethason-Phosphat (Formulierung B, C, je 3,75 mg/kg, nach Tabelle 4) behandelt. Als Kontrolle dienen freies Dexamethason-Phosphat (Formulierung K, ebenfalls 3,75 mg/kg) und physiologische Salzlösung (Saline). Die Wirkung der Probengabe wurde anhand folgender Parameter ermittelt:
- Bestimmung der Gelenkschwellung (vgl. Figur 5)
- histologische Begutachtung von Entzündungsparametern und Parametern der Gelenkknorpelzerstörung (vgl Figur 2 und Tabelle 5)

**Tabelle 5**

| Formulierung | Grad der Knorpelzerstörung | Grad der Gelenkentzündung |
|---|---|---|
| Saline | 4 , 0 | 5 , 5 |
| Formulierung K | 2,75 | 3,5 |
| Formulierung B | 0 | 0,25 |
| Formulierung C | 1,0 | 1,5 |

Grad der Gelenkknorpelzerstörung und Gelenkentzündung der arthritischen Tiere nach Behandlung
0 - keine Veränderungen im Vergleich zum gesunden Knie
6 - starke Veränderungen im Vergleich zum gesunden Knie

Sowohl Gelenkknorpelzerstörung als auch Entzündungsreaktionen werden durch Behandlung mit liposomalem Dexamethason-Phosphat wirksam verringert. Freier Wirkstoff in der angegebenen Konzentration ist erheblich schlechter wirksam.

### Beispiel 9

### Dosisreduktion

Eine Arthrits wurde analog Beispiel 8 an Ratten ausgelöst und diese mit folgenden Formulierungen behandelt, die nur ein Zehntel der in Beispiel 8 angegebenen Dosis Dexamethason-Phosphat enthielten:
Formulierung B-25 µg = 0,375 mg/ kG KG
Formulierung K 25 µg = 0,375 mg/ kG KG

Die gemessenen Gelenkschwellungen werden auch in der Figur 5 dargestellt. Ein fast gleicher therapeutischer Effekt ist auch mit der verringerten Dosis festzustellen, die Gelenkschwellung bildet sich bei Formulierung B nach etwa 3 Tagen vollständig zurück, was bei Formulierung K nicht auftritt.

### Figurenbeschreibung

### Figur 1

Gelenkschwellung der arthritischen Tiere über den Untersuchungszeitraum von 21 Tagen nach Auslösung der Arthritis im rechten Knie im Vergleich zum gesunden linken Knie

### Figur 2

Histologische Schnitte der arthritischen Kniegelenke. Hämatoxylin/Eosin-Färbung, 92fache Vergrößerung
K - Formulierung K, starke Entzündung und Knorpel- und Knochendestruktion
A - Formulierung A, geringfügig entzündliche Infiltration
C - Formulierung C, keine Entzündungsreaktion

### Figur 3

Infrarotfluoreszenz einer Ratte, die mit Cy5.5^{™}-BSA gefüllten Liposomen der Formulierung DPPC/DPPG/Chol 50:10:40 behandelt wurde.

### Figur 4

Vergleich der zeitlichen Anreicherung der Liposomen im arthrithischen zum nicht arthrithischen Gelenk.

### Figur 5

Gelenkschwellung der arthritischen Tiere über den Untersuchungszeitraum von 21 Tagen nach Auslösung der Arthritis im rechten Knie im Vergleich zum gesunden linken Knie:
Formulierung 3 HisChol = Formulierung B
Formulierung 5 DOTAP = Formulierung C
Formulierung DXM-Phosphat = Formulierung K

### Abkürzungen:

| | |
|---|---|
| HistChol | Nα-Histidinyl-Cholesterolhemisuccinat |
| DOTAP | N-[1-(2,3-Dioleoyloxy)propyl]-N,N,N-trimethylammonium |
| MoChol | 4-(2-Aminoethyl)-Morpholino-Cholesterolhemisuccinat |
| HisChol | Histaminyl-Cholesterolhemisuccinat |
| AC | Palmitoylcarnosin (Acylcarnosin) |
| Chems | Cholesterylhemisuccinat |
| DPPC | Dipalmitoylphosphatidylcholin |
| DPPG | Dipalmitoylphosphatidylglycerol |
| POPC | Palmitoyloleoylphosphatidylcholin |
| Chol | Cholesterol |
| BSA | Rinderserumalbumin |

### Literatur

Mizushima, Y.; Hamano, T.; Yokoyama, K. (1982): Tissue distribution and anti-inflammatory activity of corticosteroids incorporated in lipid emulsion. Ann Rheum Dis; 41(3) : 263-267
Yokoyama, K.; Okamoto, H.; Watanabe, M.; Suyama, T.; Mizushima, Y. (1985): Development of a corticosteroid incorporated in lipid microspheres (liposteroid). Drugs Exp Clin Res; 11 (9) : 611-620
Bonanomi, M.H.; Velvart, M.; Weder, H.G. (1987): Fate of different kinds of liposomes containing dexamethasome palmitate after intra-articular injection into rabbit joints. J Microencapsul; 4(3):189-200
Loftsson, T. & Stefansson, E. (2002) : Cyclodextrins in eye drop formulations : enhanced topical delivery of corticosteroids to the eye. Acta Ophthalmol Scand; 80(2): 144-150
Yano, H. ; Hirayama, F. ; Kamada, M.; Arima, H.; Uekama, K. (2002): Colon-specific delivery of prednisolone-appended alpha-cyclodextrin conjugate: alleviation of systemic side effect after oral administration. J Control Release; 79(1-3): 103-112
Storm, G. & Richmond, P.L. (2001): Pegylated liposomal doxorubicin: tolerability and toxicity. Pharmacotherapy; 21 (6) : 751-763
Metselaar, J.M. ; Wauben, M.H.M.; Boerman, O.C.; van Lent, P.L.; Storm, G. (2002): Long-circulating liposomes for i.v. targeted delivery of glucocorticoids in arthritis. Cell Mol Biol Lett; 7(2): 291-292
Hussein, M.A.; Wood, L.; His, E.; Srkalovic, G.; Karam, M.; Elson, P.; Bukowski, R.M. (2002): A phase II trial of pegylated liposomal doxorubicin, vincristine, and reduceddose dexamethasone combination therapy in newly diagnosed multiple myeloma patients. Cancer; 95(10): 2160-2168
Dams, E.T.M.; Laverman, P.; Oyen, W.J.G.; Storm, G.; Scherphof, G.; van der Meer, J.W.M.; Corstens, F.H.M.; Boerman, O.C. (2000): Accelerated blood clearance and altered biodistribution of repeated injections of sterically stabilized liposomes. The Journal of Pharmacology and Experimental Therapeutics, 292(3): 1071-1079
EP 1 046 394 (Terumo Corp.)
WO 02/45688 (Yamanouchi Euro BV): Parenteral compositions for site-specific treatment of inflammatory disorders comprises liposomes composed of non-charged vesicle-forming lipids and a water soluble corticosteroid
WO 95/15746 (Univ. London School Pharmacy): New liposomes, esp. For drug delivery - having an internal aq. Phase cont. Complex of active cpd. With receptor, e.g. for rendering hydrophobic drugs hydrophilic
WO 94/07466 (Liposome Technology Inc.): Treatment of inflamed tissue - using liposomes contg. a lipid derivatised with polyethylene glycol entrapping a therapeutic cpd.
DE 27 12 030 (Imperial Chem Ind., 1977) : Antiinflammatory medicaments based on liposomes - contg. a lipophilically subst. steroid

## Patentansprüche

1. Liposomale Formulierung, **dadurch gekennzeichnet, dass** in der inneren wässrigen Phase der Liposomen wasserlösliche Glucocorticoide vorliegen und dass die Liposomen eine Größe zwischen 150 und 500 nm besitzen, bei physiologischem pH von 7,5 einen Überschuss an negativer Ladung aufweisen und
a) aufgebaut aus gesättigten Phospholipiden, einen Anteil an negativen Ladungsträgern von nicht mehr als 20 mol% und einen Cholesterolgehalt zwischen 35 und 45 mol% der Liposomenmenbran aufweisend,
oder
b) amphotere Liposomen sind,
und dass die Liposomen kein amphiphil modifiziertes Polyethylenglykol umfassen.

2. Liposomale Formulierung nach Anspruch 1, **dadurch gekennzeichnet, dass** der Anteil negativer Ladungsträger zwischen 5 und 15 mol% liegt.

3. Liposomale Formulierung nach Anspruch 1, **dadurch gekennzeichnet, dass**
die Liposomenmembran der amphoteren Liposomen neutrale, kationische und anionische Lipide enthält und dass der molare Anteil der negativen Ladungsträger nicht mehr als 40 mol%, besonders bevorzugt 5 - 30 mol%, der der positiven Ladungsträger nicht mehr als 40 mol%, besonders bevorzugt 5 - 30 mol % und der der neutralen Gerüstlipide 30 - 80 mol%, besonders bevorzugt 40 - 70 mol% umfasst.

4. Liposomale Formulierung nach Anspruch 1,
**dadurch gekennzeichnet, dass**
die Liposomenmembran der amphoteren Liposomen neutrale und amphotere Lipide enthält und der molare Anteil der neutralen Gerüstlipide zwischen 30 und 80 mol%, besonders bevorzugt zwischen 40 und 70 mol% umfasst.

5. Liposomale Formulierung nach einem der Ansprüche 1 bis2,
**dadurch gekennzeichnet, dass**
die gesättigten Phospholipide aus der Gruppe Dipalmitoylphosphytidylcholin, Distearoylphosphytidylcholin, Dipalmitoylphosphatidylglycerol, Distearoylphosphatidylglycerol, Dipalmitoylphosphatidylserin, Distearoylphosphatidylserin ausgewählt werden.

6. Liposomale Formulierung nach Anspruch 3 oder 4, **dadurch gekennzeichnet, dass**
die neutralen Gerüstlipide aus der Gruppe Dimyristoylphosphytidylcholin, Dipalmitoylphosphytidylcholin, Palmitoyloleoylphosphatidylcholin, Distearoylphosphytidylcholin und/oder Cholesterol ausgewählt werden, oder gereinigte Cholinfraktionen natürlichen Ursprungs wie Soja-Phosphatidylcholin oder Ei-Phosphatidylcholin umfassen.

7. Liposomale Formulierung nach einem der Ansprüche 1, 2 oder 5 , **dadurch gekennzeichnet, dass** die negativen Ladungsträger Phosphatidylglycerol und Phosphatidylserin, besonders bevorzugt Phosphatidylglycerol, ganz besonders bevorzugt Dipalmitoylphosphatidylglycerol.

8. Liposomale Formulierung nach einem der Ansprüche 1 bis 7,
**dadurch gekennzeichnet, dass**
die wasserlöslichen Glucocorticoide deren
Phosphatester, Dicarbonsäureester, Additionssalze, Glycoside oder Sulfatester sind.

9. Liposomale Formulierung nach Anspruch 8, **dadurch gekennzeichnet, dass**
das wasserlösliche Glucocorticoid aus der Gruppe Dexamethasonphosphat, Dexamethasondihydrogenphosphat-Dinatrium, Triamcinoloneacetonidphosphat Prednisolonphosphat ausgewählt ist.

10. Verwendung der liposomalen Formulierung nach einem der Ansprüche 1 bis 9 zur Herstellung eines pharmazeutischen Präparates zur systemischen Applikation.

11. Verwendung der liposomalen Formulierung nach einem der Ansprüche 1 bis 9 zur Herstellung eines pharmazeutischen Präparates zur Behandlung von inflammatorischen Erkrankungen.

12. Verwendung einer liposomalen Formulierung zur Herstellung eines pharmazeutischen Präparates,
**dadurch gekennzeichnet, dass**
in der inneren wässrigen Phase der Liposomen wasserlösliche Glucocorticoide vorliegen und dass die Liposomen keine amphiphil modifizierten Polymere wie Polyethylenglykol-Phosphatidylethanolamin umfassen, zur systemischen Applikation bei Mensch oder Tier zur Therapie von rheumatischer Arthritis.

13. Verwendung der liposomalen Formulierung nach einem der Ansprüche 1 bis 9 zur Herstellung eines pharmazeutischen Präparates zur systemischen Applikation bei Mensch oder Tier zur Therapie von inflammatorischen Krankheiten.

14. Verwendung der liposomalen Formulierung, nach einem Ansprüche 1 bis 9 zur Herstellung eines pharmazeutischen Präparates zur systemischen Applikation bei Mensch oder Tier zur Therapie von rheumatischer Arthritis.

## Claims

1. A liposomal formulation, **characterized in that** water-soluble glucocorticoids are present in the interior aqueous phase of the liposomes, and that the liposomes have a size between 150 and 500 nm,
a) formed of saturated phospholipids, comprising a proportion of negative charge carriers no higher than 20 mole-% and a cholesterol content of the liposome membrane between 35 and 45 mole-%,
or
b) being amphoteric liposomes
and that the liposomes do not comprise any amphiphilic-modified polyethylene glycol.

2. The liposomal formulation according to claim 1,
**characterized in that**
the molar proportion of negative charge carriers is between 5 and 15 mole-%.

3. The liposomal formulation according to claim 1,
**characterized in that**
the liposome membrane of the amphoteric liposomes contains neutral, cationic and anionic lipids, and that the molar proportion of anionic charge carriers is no higher than 40 mole-%, more preferably 5 to 30 mole-%, that of cationic charge carriers no higher than 40 mole-%, more preferably 5 to 30 mole-%, and that of neutral backbone lipids 30 to 80 mole-%, more preferably 40 to 70 mole-%.

4. The liposomal formulation according to claim 1,
**characterized in that**
the liposome membrane of the amphoteric liposomes contains neutral and amphoteric lipids, and that the molar proportion of neutral backbone lipids is between 30 and 80 mole-%, more preferably between 40 and 70 mole-%.

5. The liposomal formulation according to claim 1 or 2,
**characterized in that**
the saturated phospholipids are selected from the group of dipalmitoylphosphatidyl choline,
distearoylphosphatidyl choline,
dipalmitoylphosphatidyl glycerol,
distearoyiphosphatidyl glycerol,
dipafmitoylphasphatidyl serine,
distearoylphosphatidyl serine.

6. The liposomal formulation according to claim 3 or 4,
**characterized in that**
the neutral backbone lipids are selected from the group of dimyristoylphosphatidyl choline,
dipalmitoylphosphatidyl choline,
palmitoyloleoylphosphatidyl choline,
distearoylphosphatidyl choline and/or
cholesterol, or comprise purified choline fractions of natural origin such as soy phosphatidyl choline or egg phosphatidyl choline.

7. The liposomal formulation according to any of claims 1, 2 or 5
**characterized in that**
the negative charge carriers are phosphatidyl glycerol and phosphatidyl serine, more preferably phosphatidyl glycerol and especially preferred dipalmitoy! phosphatidyl glycerol.

8. The liposomal formulation according to any of claims 1 - 7,
**characterized in that**
the water-soluble glucocorticoids are phosphate esters, dicarboxylic acid esters, addition salts, glycosides or sulfate esters of the glucocorticoids.

9. The liposomal formulation according to claim 8, **characterized in that** the water-soluble glucocorticoid is selected from the group of dexamethasone phosphate, dexamethasone dihydrogen phosphate disodium, triamcinolone acetonide phosphate, prednisolone phosphate.

10. Use of the liposomal formulation according to any of claims 1 to 9 for the production of a pharmaceutical composition for systemic application.

11. Use of the liposomal formulation according to any of claims 1 to 9 for the production of a pharmaceutical composition for the treatment of inflammatory diseases.

12. Use of a liposomal formulation for the production of a pharmaceutical composition, **characterized in that** water-soluble glucocorticoids are present in the interior aqueous phase of the liposomes, and that the liposomes do not comprise any amphiphilic-modified polymers, in systemic application in a human or animal for the therapy of rheumatic arthritis.

13. Use of the liposomal formulation according to any of claims 1 to 9 for the production of a pharmaceutical composition for systemic application in a human or animal for the therapy of inflammatory diseases.

14. Use of the liposomal formulation according to any of claims 1 to 9 for the production of a pharmaceutical composition for systemic application in a human or animal for the therapy of rheumatic arthritis.

## Revendications

1. Formulation liposomale, **caractérisée par** la présence de glucocorticoïdes hydrosolubles dans la phase aqueuse intérieure des liposomes et en ce que les liposomes ont une taille entre 150 et 500 nm, qu'ils présentent un excédent de charges négatives à un pH physiologique de 7,5 et
a) sont constitués de phospholipides saturés, ayant une teneur maximale de 20 % en moles en porteurs de charges négatives et une teneur en cholestérol entre 35 et 45 % en moles de la membrane liposomale,
ou
b) sont des liposomes amphotères,
et en ce que les liposomes ne comprennent pas de polyéthylène glycol modifié pour le rendre amphiphile.

2. Formulation liposomale selon la revendication 1, **caractérisée en ce que** la teneur en porteurs de charges négatives est comprise entre 5 et 15 % en moles.

3. Formulation liposomale selon la revendication 1, **caractérisée en ce que** la membrane liposomale des liposomes amphotères contient des lipides neutres, cationiques et anioniques et que la teneur molaire des porteurs de charges négatives est au maximum de 40 % en moles, de manière plus préférée de 5 à 30 % en moles, celle des porteurs de charges positives est au maximum de 40 % en moles, de manière plus préférée de 5 à 30 % en moles, et celle des lipides structuraux neutres est de 30 à 80 % en moles, de manière plus préférée de 40 à 70 % en moles.

4. Formulation liposomale selon la revendication 1, **caractérisée en ce que** la membrane liposomale des liposomes amphotères contient des lipides neutres et amphotères et la teneur molaire des lipides structuraux neutres est entre 30 et 80 % en moles, de manière particulièrement préférée entre 40 et 70 % en moles.

5. Formulation liposomale selon l'une quelconque des revendications 1 à 2,
**caractérisée en ce que**
les phospholipides saturés sont sélectionnés parmi le groupe comprenant la dipalmitoylphosphatidylcholine, la distéaroylphosphatidylcholine, le dipalmitoylphosphatidylglycérol, le distéaroylphosphatidylglycérol, la dipalmitoylphosphatidylsérine, et la distéaroylphosphatidylsérine.

6. Formulation liposomale selon la revendication 3 ou 4, **caractérisée en ce que** les lipides structuraux neutres sont choisis dans le groupe comprenant la dimyristoylphosphatidylcholine, la dipalmitoylphosphatidylcholine, la palmitoyloléoylphosphatidylcholine, la distéaroylphosphatidylcholine et/ou le cholestérol, ou comprennent des fractions de choline purifiées d'origine naturelle comme la phosphatidylcholine de soja ou la phosphatidylcholine d'oeuf.

7. Formulation liposomale selon l'une quelconque des revendications 1, 2 ou 5, **caractérisée en ce que**
les porteurs de charges négatives sont du phosphatidylglycérol et de la phosphatidylsérine, de préférence du phosphatidylglycérol, et de manière encore plus préférée du dipalmitoylphosphatidylglycérol.

8. Formulation liposomale selon l'une quelconque des revendications 1 à 7, **caractérisée en ce que**
les glucocorticoïdes hydrosolubles sont leurs esters de phosphate, esters d'acide dicarboxylique, sels d'addition, glycosides ou esters de sulfate.

9. Formulation liposomale selon la revendication 8, **caractérisée en ce que** le glucocorticoïde hydrosoluble est sélectionné dans le groupe comprenant le dexaméthasone phosphate, le dexaméthasone hydrogénophosphate disodique, le triamcinolone acétonide phosphate et le prednisolone phosphate.

10. Utilisation de la formulation liposomale selon l'une quelconque des revendications 1 à 9 pour préparer une préparation pharmaceutique destinée à une administration systémique.

11. Utilisation de la formulation liposomale selon l'une quelconque des revendications 1 à 9 pour préparer une préparation pharmaceutique destinée au traitement des maladies inflammatoires.

12. Utilisation d'une formulation liposomale pour préparer une préparation pharmaceutique, **caractérisée par** la présence de glucocorticoïdes hydrosolubles dans la phase aqueuse interne des liposomes et en ce que les liposomes ne comportent pas de polymères modifiés pour les rendre amphiphiles comme le polyéthylèneglycol-phosphatidyléthanolamine, destinée à une administration systémique chez l'homme ou l'animal pour la thérapie de l'arthrite rhumatoïde.

13. Utilisation de la formulation liposomale selon l'une quelconque des revendications 1 à 9 pour préparer une préparation pharmaceutique destinée à une administration systémique chez l'homme ou l'animal pour la thérapie des maladies inflammatoires.

14. Utilisation de la formulation liposomale selon l'une quelconque des revendications 1 à 9 pour préparer une préparation pharmaceutique destinée à une administration systémique chez l'homme ou l'animal pour la thérapie de l'arthrite rhumatoïde.
